# EUROPEAN PATENT SPECIFICATION

(11) **EP 2 300 444 B1**
(45) Date of publication and mention of the grant of the patent: **13.04.2016**
(21) Application number: 09749530.3
(22) Date of filing: 24.04.2009
(51) Int. Cl.: C07D 251/10

(54) **PROCESS FOR THE SYNTHESIS OF 3,6-DIHYDRO-1,3,5-TRIAZINE DERIVATIVES**
VERFAHREN ZUR SYNTHESE VON 3,6-DIHYDRO-1,3,5-TRIAZINDERIVATEN
PROCÉDÉ POUR LA SYNTHÈSE DE DÉRIVÉS DE 3,6-DIHYDRO-1,3,5-TRIAZINE

(30) Priority: 23.05.2008 EP 08009483
(43) Date of publication of application: 30.03.2011
(73) Proprietor: POXEL SAS, 69007 Lyon (FR)
(72) Inventor: AUBOURG, Stéphanie, F- 45800 Saint Jean de Braye (FR); BOUDET, Bernard, F-45300 Pithiviers (FR); CRAVO, Daniel, F-78500 Sartrouville (FR); HELMREICH, Matthias, 69120 Heidelberg (DE)
(74) Representative: Tezier Herman, Béatrice
(86) International application number: PCT/EP2009/002997
(87) International publication number: WO 2009/141040

(56) References cited:
- WO-A-01/55122
- WO-A-2009/062483

## Description

3,6-Dihydro-1,3,5-triazine derivatives show pharmacological properties in the treatment of pathological conditions associated with the insulin-resistance syndrome. Several patents describe the preparation of 3,6-dihydro-1,3,5-triazine derivatives. For example, in US3287366 the synthesis of dihydro-triazine bearing the following structure is described:

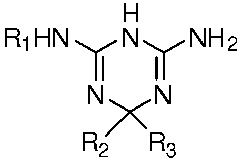

The synthesis involves the reaction of a mono-substituted bisguanidine and an aldehyde or ketone in presence of an acid at elevated temperatures.

Patent JP48064088 describes the synthesis of dihydro-triazines bearing the following structure:

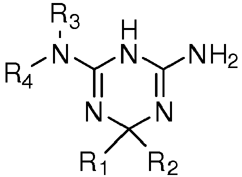

The analogous synthesis also involves heating under acidic condition.

Patent JP54014986 describes the synthesis of dihydro-triazines bearing the following structure: 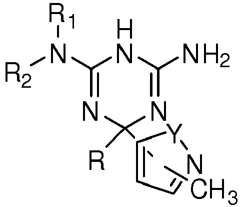 Similarly, this method requires heating under acidic conditions.

Patent application WO 01/55122 describes the synthesis of dihydro-triazines of the following structure:

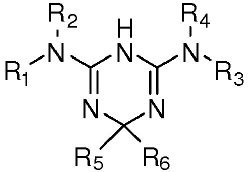

The synthesis is directed to the reaction of mono-substituted bisguanidines and an acetal, hemiacetal, ketal, hemiketal, aldehyde, or ketone in presence of an acid at elevated temperatures.

Common to the published procedures is the requirement of elevated temperature, which may require refluxing conditions or high pressure if low boiling point starting materials are employed as well as the use of an acid. The invention had the object of finding a new reaction under ambient conditions while only inexpensive starting materials are used.

This would save energy and improve the safety of the process.

Unexpectedly, it has been found, that compounds of formula I can be prepared in presence of a base selected from the group K₂CO₃, NaHCO₃, NaOMe, Na₂CO₃, piperidine and morpholine, preferably at temperatures between -10° (preferably -5 °) and 80 °C under ambient pressure.

More preferably, the reaction is carried out at temperatures between -5 ° and 60 °C and most preferably at temperatures between -5° and 20°C, under ambient pressure.

The invention relates to a process for the preparation of compounds of the formula I 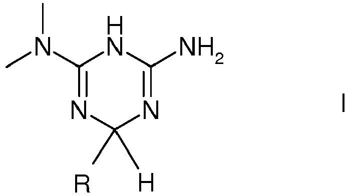 in which
R is methyl, phenyl, 4-hydroxy-phenyl or 4-methoxyphenyl and pharmaceutically salts, tautomers and stereoisomers thereof, characterised in that a compound of the formula II 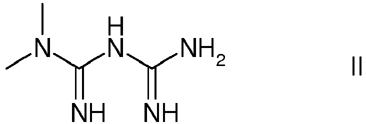 and the salts thereof
is reacted with a compound of the formula III

   R-CHO III
in which R is as defined above,
in a polar solvent or solvent mixture in presence of an inorganic and/or organic base, wherein the base is selected from the group consisting of K₂CO₃, NaHCO₃, NaOMe, Na₂CO₃, piperidine and morpholine.

The solvent may be chosen from water, methanol, ethanol, isopropanol, n-butanol, 2-butanol, i-butanol, t-butanol, N,N-dimethyl formamide or any combination thereof.

The base particularly preferred is NaOMe or piperidine.

The solvent particularly preferred is methanol, isopropanol or a mixture of water and methanol.

The concentration of the compound of formula II ranges from 0.1 mol/L to 4 mol/L. The concentration of the compound of formula III ranges from 1 equivalent to 10 equivalents to the compound of formula II.

The base ranges from 0.5 equivalent to 10 equivalents, preferably from 0.9 equivalent to 10 equivalents, to the compound of formula II.

The compounds of formula I also mean their solvates and their pharmaceutically usable derivatives.
The term "solvates of the compounds" is taken to mean adductions of inert solvent molecules onto the compounds which form owing to their mutual attractive force. Solvates are, for example, mono- or dihydrates or alcoholates.

The term "pharmaceutically usable derivatives" is taken to mean, for example, the salts of the compounds according to the invention and so-called prodrug compounds.
The term "prodrug derivatives" is taken to mean compounds of the formula I which have been modified with, for example, alkyl or acyl groups, sugars or oligopeptides and which are rapidly cleaved in the organism to form the active compounds according to the invention.

These also include biodegradable polymer derivatives of the compounds according to the invention, as described, for example, in Int. J. Pharm. 115, 61-67 (1995).

Formula I also embraces the tautomeric forms of the compounds.

Tautomeric forms of the compound of formula I in which R is methyl:

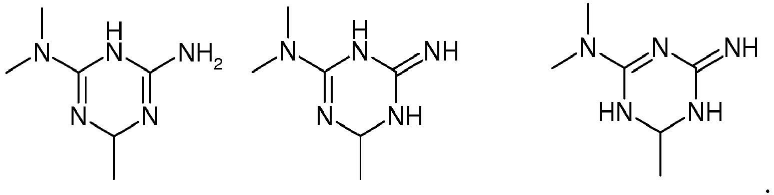

A preferred process for the preparation of compounds of formula I is related to compounds
in which
R is methyl or 4-hydroxyphenyl; most preferably R is methyl.

Most preferably the reaction is carried with NaOMe as base in methanol at temperatures between -5 ° and 20°C.

Most preferred is the process for the preparation of the compound of formula I 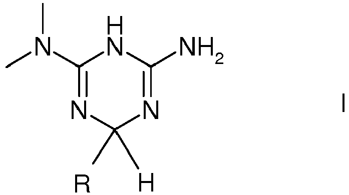 in which
R is methyl
and pharmaceutically salts, tautomers and stereoisomers thereof, characterised in that a compound of the formula II

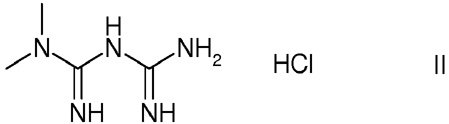

is reacted with a compound of the formula III

   R-CHO III
in which R is as defined above,
in a polar solvent or solvent mixture in presence of an inorganic and/or organic base, wherein the base is selected from the group consisting of K₂CO₃, NaHCO₃, NaOMe, Na₂CO₃, piperidine and morpholine.

There is an example giving further detail on the invention, but the invention is not limited within the example.

### Example 1:

Preparation of 2-amino-3,6-dihydro-4-dimethylamino-6-methyl-1,3,5-triazine:
A suspension of 10 g (60 mmol) N,N-dimethylbiguanide hydrochloride in 50 ml methanol is cooled to 0 to -5°C. 5.3 g (120 mmol) acetaldehyde and 10.9 g (60 mmol) NaOMe solution (30 % in methanol) are added. HPLC after 20 hours shows conversion to the desired product;
¹H NMR (300 MHz, CDCl₃) δ 1.37 (d, *J* = 6.0 Hz, 3H), 2.98 (s, 6H), 3.46 (s, 1H), 4.83 (q, *J* = 6.0 Hz, 1H). ¹³C NMR (300 MHz, CDCl₃) δ 24.5, 36.4, 63.0.

### Example 2:

Preparation of 2-amino-3,6-dihydro-4-dimethylamino-6-(4-hydroxyphenyl)-1,3,5-triazine:
A suspension of 30.42 g (0.235 mol) N,N-dimethylbiguanide, 30.2 g (0.246 mol) para-hydroxybenzaldehyde and 3 ml piperidine in 300 ml isopropanol is stirred at reflux for 20 hours. Upon cooling, a solid precipitates, which is recovered and washed with cooled isopropanol;
yield: 42 g 2-amino-3,6-dihydro-4-dimethylamino-6-(4-hydroxyphenyl)-1,3,5-triazine.

## Claims

1. Process for the preparation of compounds of formula I 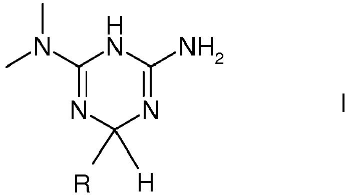 in which
R is methyl, phenyl, 4-hydroxy-phenyl or 4-methoxyphenyl
and pharmaceutically salts, tautomers and stereoisomers thereof, **characterised in that** a compound of the formula II 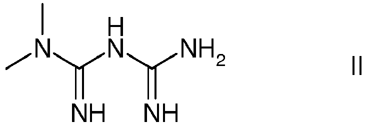
and the salts thereof
is reacted with a compound of the formula III
R-CHO III
in which R is as defined above,
in a polar solvent or solvent mixture in presence of an inorganic and/or organic base selected from the group consisting of K₂CO₃, NaHCO₃, NaOMe, Na₂CO₃, piperidine, and morpholine.

2. Process according to claim 1, in which R is methyl or 4-hydroxyphenyl.

3. Process according to anyone of claims 1 and 2, in which the base is chosen from the group of NaOMe or piperidine.

4. Process according to one of claims 1-3, in which the solvent is chosen from the group as single solvent or solvents mixture: water, methanol, ethanol, isopropanol, n-butanol, 2-butanol, i-butanol, t-butanol, N,N-dimethyl formamide.

5. Process according to one of claims 1-4, in which the concentration of compound of formula II is from 0.1 mol/L to 4 mol/L.

6. Process according to one of claims 1-5, in which the compound of formula III is from 1 equivalent to 10 equivalents to the compound of formula II.

7. Process according to one of claims 1-6, in which the base is from 0.9 equivalent to 10 equivalents to the compound of formula II.

8. Process according to one of claims 1-7, in which the compound of formula III is acetaldehyde.

9. Process according to one of claims 1-8, in which the reaction is performed at temperatures between -10 and 80°C under ambient pressure.

10. Process according to one of claims 1-9, in which the reaction is performed at temperatures between -5 and 60°C under ambient pressure.

11. Process according to one of claims 1-10, in which the reaction is performed at temperatures between -5 and 20°C under ambient pressure.

## Patentansprüche

1. Verfahren für die Herstellung von Verbindungen nach Formel I 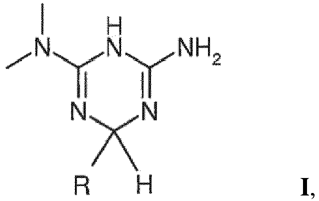 in welcher
R Methyl, Phenyl, 4-Hydroxy-phenyl oder 4-Methoxyphenyl ist
und pharmazeutische Salze, Tautomere und Stereoisomere davon, dadurch charakterisiert, dass eine Verbindung der Formel II 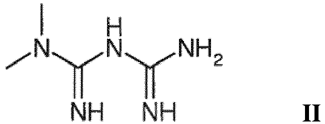
und Salze davon
mit einer Verbindung der Formel III reagiert,
R-CHO **III,**
in welcher R wie oben definiert ist,
in einem polaren Lösungsmittel oder Lösungsmittelgemisch in Gegenwart einer anorganischen und/oder organischen Base ausgewählt aus der Gruppe bestehend aus K₂CO₃, NaHCO₃, NaOMe, Na₂CO₃, Piperidin und Morpholin.

2. Verfahren nach Anspruch 1, in welchem R Methyl oder 4-Hydroxyphenyl ist.

3. Verfahren nach einem der Ansprüche 1 und 2, in welchem die Base ausgewählt ist aus der Gruppe von NaOMe oder Piperidin.

4. Verfahren nach einem der Ansprüche 1 - 3, in welchem das Lösungsmittel ausgewählt ist aus der Gruppe als Einzellösungsmittel oder Lösungsmittelgemisch: Wasser, Methanol, Ethanol, Isopropanol, n-Butanol, 2-Butanol, i-Butanol, t-Butanol, N,N-Dimethylformamid.

5. Verfahren nach einem der Ansprüche 1 - 4, in welchem die Konzentration der Verbindung nach Formel II von 0,1 mol/L bis 4 mol/L ist.

6. Verfahren nach einem der Ansprüche 1 - 5, in welchem die Verbindung nach Formel III von 1 Äquivalent bis 10 Äquivalenten zu der Verbindung nach Formel II ist.

7. Verfahren nach einem der Ansprüche 1 - 6, in welchem die Base von 0,9 Äquivalenten bis 10 Äquivalenten zu der Verbindung nach Formel II ist.

8. Verfahren nach einem der Ansprüche 1 - 7, in welchem die Verbindung nach Formel III Acetaldehyd ist.

9. Verfahren nach einem der Ansprüche 1 - 8, in welchem die Reaktion bei Temperaturen zwischen -10 und 80°C unter Umgebungsdruck durchgeführt wird.

10. Verfahren nach einem der Ansprüche 1 - 9, in welchem die Reaktion bei Temperaturen zwischen -5 und 60°C unter Umgebungsdruck durchgeführt wird.

11. Verfahren nach einem der Ansprüche 1 - 10, in welchem die Reaktion bei Temperaturen zwischen -5 und 20°C unter Umgebungsdruck durchgeführt wird.

## Revendications

1. Procédé de préparation de composés de formule I 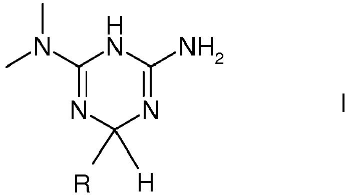 dans laquelle
R est méthyle, phényle, 4-hydroxy-phényle ou 4-méthoxyphényle
et leurs sels pharmaceutiques, tautomères et stéréoisomères,
**caractérisé en ce qu'**un composé de formule II 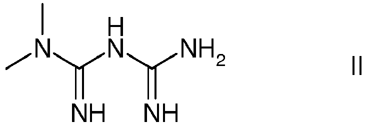
et ses sels
est mis à réagir avec un composé de formule III
R-CHO III
dans laquelle R est tel que défini ci-dessus,
dans un solvant polaire ou un mélange de solvants polaires, en présence d'une base inorganique et/ou organique choisie dans le groupe constitué de K₂CO₃, NaHCO₃, NaOMe, Na₂CO₃, pipéridine, et morpholine.

2. Procédé selon la revendication 1, dans lequel R est méthyle ou 4-hydroxyphényle.

3. Procédé selon l'une quelconque des revendications 1 et 2, dans lequel la base est choisie dans le groupe de NaOMe ou la pipéridine.

4. Procédé selon l'une quelconque des revendications 1 à 3, dans lequel le solvant est choisi dans le groupe de: l'eau, le méthanol, l'éthanol, l'isopropanol, le n-butanol, le 2-butanol, l'i-butanol, le t-butanol, la N,N-dimethyl formamide, sous forme de solvants uniques ou de mélanges de solvants.

5. Procédé selon l'une quelconque des revendications 1 à 4, dans lequel la concentration du composé de formule II va de 0,1 mol/L à 4 mol/L.

6. Procédé selon l'une quelconque des revendications 1 à 5, dans lequel le composé de formule III va de 1 équivalent à 10 équivalents par rapport au composé de formule II.

7. Procédé selon l'une quelconque des revendications 1 à 6, dans lequel la base va de 0,9 équivalent à 10 équivalents par rapport au composé de formule II.

8. Procédé selon l'une quelconque des revendications 1 à 7, dans lequel le composé de formule III est l'acétaldéhyde.

9. Procédé selon l'une quelconque des revendications 1 à 8, dans lequel la réaction est effectuée à des températures comprises entre -10 et 80°C sous pression ambiante.

10. Procédé selon l'une quelconque des revendications 1 à 9, dans lequel la réaction est effectuée à des températures comprises entre -5 et 60°C sous pression ambiante.

11. Procédé selon l'une quelconque des revendications 1 à 10, dans lequel la réaction est effectuée à des températures comprises entre -5 et 20°C sous pression ambiante.
